# EUROPÄISCHE PATENTSCHRIFT

(11) **EP 1 782 824 B1**
(45) Veröffentlichungstag und Bekanntmachung des Hinweises auf die Patenterteilung: **12.05.2010**
(21) Anmeldenummer: 07101239.7
(22) Anmeldetag: 06.07.2005
(51) Int. Cl.: A61K 38/44, A23L 1/305, A61K 9/06, A61K 9/50, A61P 17/00, A61P 17/04, A61P 37/00

(54) **Pharmazeutische Zubereitungen als Hydrogel, die Diaminooxidase enthalten**
Pharmaceutical preparations as hydrogel containing diaminooxidase
Compositions pharmaceutiques sous forme d'hydrogel contenant de la diamino-oxydase

(30) Priorität: 07.07.2004 AT 11502004
(43) Veröffentlichungstag der Anmeldung: 09.05.2007
(62) Teilanmeldung aus: 05758617.4
(73) Patentinhaber: Sciotec Diagnostic Technologies GmbH, 3430 Tulln (AT)
(72) Erfinder: Missbichler, Albert, 1210, Wien (AT); Reichl, Herwig, 8262, Ilz (AT); Gabor, Franz, 3385, Gerersdorf (AT)
(74) Vertreter: Sonn & Partner Patentanwälte

(56) Entgegenhaltungen:
- EP-A- 0 132 674
- WO-A-02/066669
- FR-A- 2 101 095
- FR-A- 2 215 944
- US-A- 3 639 579
- US-A- 3 721 733
- US-A- 4 652 449
- US-A- 4 725 540
- US-A- 5 270 033
- US-A1- 2004 115 189

## Beschreibung

Die vorliegende Erfindung betrifft Zusammensetzungen zur Behandlung bzw. zur Vorbeugung von Histamin-induzierten Krankheiten und Krankheitszuständen.

Histamin (1H-Imidazol-4-Ethylamin) entsteht durch enzymatische Decarboxylierung von Histidin und ist somit ein basisches biogenes Amin mit einem Molekulargewicht von 111 Da.

Im Organismus kommt Histamin praktisch ubiquitär vor. Es wird vom Menschen selbst produziert und in den metachromatischen Granula der Mastzellen und basophilen Leukozyten in inaktiver Form gespeichert, wo es zur sofortigen Freisetzung zur Verfügung steht. Die höchsten Histamin-Konzentrationen werden in der Lunge gemessen. Nach der Freisetzung ist Histamin ein äußerst potenter Mediator einer Vielzahl von pyhsiologischen und pathophysiologischen Prozessen, oftmals auch über Wechselwirkung mit Zytokinen.

Darüber hinaus kann Histamin auch von außen in den Körper gelangen, einerseits durch Einatmen oder aber auf dem oralen Weg, beispielsweise durch die Aufnahme von histaminhältigen Lebensmitteln, wie Käse, Wein, Fischkonserven und Sauerkraut.

Die wichtigsten Funktionen und Wirkungen von Histamin im menschlichen bzw. tierischen Körper sind:
1)Dilatation der Kapillaren, Erhöhung der Kapillarpermeabilität und Blutdruckabfall.
2)Kontraktionen der glatten Muskulatur, u.a. der Bronchialmuskeln in der Lunge.
3)Induktion einer erhöhten Magensäuresekretion.
4)Erhöhung der Herzfrequenz.
5)Histamin ist der Mediator der allergischen Soforttyp-Reaktion, es ist der wichtigste Mediator bei allergischen Erkrankungen, wie Rhinitis allergica (Heuschnupfen) und Asthma bronchiale.
6)Darüber hinaus ist Histamin der klassische Auslöse einer Urticaria (Nesselausschlag) und spielt bei Medikamenten-Allergien bzw. Unverträglichkeiten eine wichtige Rolle.

Hohe Konzentrationen von freiem zirkulierendem Histamin lösen unerwünschte Wirkungen aus wie Kopfschmerzen, verlegte bzw. rinnende Nase, Atemwegsobstruktionen, Tachykardie sowie Extrasystolen, weiters Magen- und Darmbeschwerden, die zu weichem Stuhl bis Durchfällen führen können, und Hypotonie. Oft werden auch Schwellungen der Augenlider, gelegentlich auch urticarielle Exantheme beschrieben. Des Weiteren können Hautrötungen, Blutdruckabfall und Bronchospasmen auftreten. Folgende Tabelle zeigt die Symptome in Abhängigkeit von der Histaminkonzentration im Blut.

| **Histamin (ng/ml)** | **Körperreaktionen** |
|---|---|
| 0-1 | keine |
| 1-2 | Verstärkte Magensaftsekretion |
| 3-5 | Tachykardie, Hautirritationen |
| 6-8 | Blutdruckabfall |
| 7-12 | Bronchospasmus |
| approx.100 | Herzstillstand |

Im Organismus von Säugetieren wird Histamin von zwei Enzymen abgebaut: Diaminooxidase (DAO, EC 1.4.3.6) und Histamin-N-Methyltransferase (NMT, EC 2.1.1.8) (Mizuguchi et al. 1994). DAO katalysiert die oxidative Deaminierung von Histamin zu Imidazolazetaldehyd; NMT katalysiert die N-methylierung zu N-Methylhistamin.

Beide Abbauwege sind für den Organismus essentiell: DAO entfernt Histamin, das beispielsweise über die Nahrung in den Gastrointestinaltrakt aufgenommen wurde, NMT steuert die histaminerge Signalübertragung im Nervensystem (Kitanaka et al. 2002).

Die Hauptaufgabe der DAO ist es zu verhindern, dass über die Nahrung aufgenommenes Histamin aus dem Darm in den Blutkreislauf gelangt. Versagt dieser Schutzmechanismus, kann es im Extremfall auch zu einem anapyhlaktischen Schock kommen (Taylor 1986, Nilsson et al. 1996). DAO ist ein sekretorisches Protein und arbeitet daher extrazellulär, wohingegen N-Methyltransferase ausschließlich im Zytosol aktiv ist (Küfner et al. 2001).

Die native DAO, die neben Histamin weitere biogene Amine, wie beispielsweise Putrescin, Spermidin und Kadaverin, abbauen kann, und die beispielsweise aus Schweineniere gewonnen wird, ist ein homodimeres kupferhältiges Glycoprotein, bei welchem die Untereinheiten über Disulfidbrücken verbunden sind. DAO hat ein Molekulargewicht von etwa 182 kDa (Kluetz and Schmidt 1997, Rinaldi et al. 1982) und einen Kohlenhydratanteil von etwa 11% (Shah and Ali 1988). Das Enzym gehört zur Klasse der kupferhältigen Aminooxidasen, welche die oxidative Deaminierung von primären Aminen zu Aldehyden, Ammoniak und Wasserstoffperoxid nach dem folgenden allgemeinen Reaktionsschema katalysieren (Bachrach 1985) : RCH₂NH₂ + H₂O + O₂ => RCHO + NH₃ + H₂O₂, wobei der Rest R eine Aminogruppe enthält.

Charakteristisch für die kupferhältigen Aminooxidasen ist ein Topachinon im aktiven Zentrum, das durch post-translationale Modifikation eines konservierten Tyrosin-Rests entsteht (James et al. 1990, James et al. 1992, Mu et al. 1992).

DAO ist hauptsächlich im Dünndarm, in der Leber, in den Nieren und im Blut in weißen Blutzellen zu finden. Bei Schwangeren wird DAO zusätzlich in der Plazenta gebildet. Schwangere haben etwa 500- bis 1000-mal höhere Blut-DAO-Spiegel als Nicht-Schwangere. DAO wird kontinuierlich produziert und in das Darmlumen ausgeschieden. Bei einem gesunden Menschen wird Histamin-reiche Nahrung daher bereits im Darm von Histamin weitgehend befreit. Das verbleibende Histamin wird beim Durchtritt durch die Darmschleimhaut von der dort befindlichen DAO abgebaut. Histamin wird zu Imidazolacetaldehyd und weiters zu Imidazol-acetessigsäure zerlegt. Die Co-Faktoren der DAO sind 6-Hydroxydopa und wahrscheinlich Pyridoxalphosphat, das Vitamin B6. DAO ist ein empfindliches Enzym, das von verschiedenen Substanzen, wie anderen biogenen Aminen, Alkohol und seinem Abbauprodukt Acetaldehyd und verschiedenen Medikamenten, gehemmt werden kann. In neuronalem Gewebe konnte bisher keine DAO-Aktivität nachgewiesen werden.

Wie bereits zuvor erwähnt kann über die Nahrung aufgenommenes, körperfremdes Histamin aber auch körpereigenes Histamin aufgrund von allergischen Reaktionen eine Vielfalt von Störungen auslösen. Hinsichtlich der klinischen Wertigkeit sind dabei mindestens drei Formen einer Histamin-Intoleranz auf der Basis einer verminderten DAO-Aktivität hervorzuheben:
- Wenige Menschen haben einen angeborenen DAO-Mangel und verlieren diesen auch nicht.
- Während eines Infektes der Darmschleimhaut kann ein vorübergehender DAO-Mangel auftreten. Nach Abheilen des Infektes normalisiert sich auch die DAO-Aktivität.
- Im Rahmen der Gabe verschiedener aktivitätshemmender Substanzen kann es exogen zu einer verminderten DAO-Aktivität kommen. Dazu gehören vorrangig Alkohol und sein Abbauprodukt Acetaldehyd, gewisse aminreiche Nahrungsmittel und kommen. Dazu gehören vorrangig Alkohol und sein Abbauprodukt Acetaldehyd, gewisse aminreiche Nahrungsmittel und viele Medikamente.

In allen Fällen treten die eingangs beschriebenen Symptome mehr oder weniger massiv auf und lassen sich in den meisten Fällen nicht einfach zuordnen. Eine schnelle Abklärung der funktionellen Aktivität des Enzyms erlaubt eine rasche und einfache Therapie und die Erstellung eines entsprechenden Diätplans.

Ein häufiger Grund für das Auftreten von Histamin-Intoleranz ist die Sensibilität des Enzyms gegenüber einer Vielzahl von chemischen Substanzen. Viele davon kommen in verschiedenen Arzneimitteln vor. Die wichtigsten DAO-Inhibitoren sind Acriflavine, Diazepam, N-Methyl-N-Formylhydrazin, b-Aminopropionitrile, Dimaprit, O-Methylhydroxylamin, Agmantine, Ethanol (10%), Pargylin, Aldomet, Furosemid, Phenamil, Amilorid, Guanabenz, Phenelzin, Aminoguanidin, Guanfacin, Phenformin, Amitryptilin, Guanidin, Phenyprazin, Amodiaquin, Haloperidol, Promethiazin, Anserin, Hyamin 1622, Propranolol, Aziridinylalkylamine, Hydroxychloroquin, B1 Pyrimidin, Hydroxylamin, Quinacrin, Burimamid, Impromidin, Semicarbazid, Cadaverin, Imidazolderivate, Thiamin, Carnosin, Iproniazid, Thioridazin, Chlorothiazid, Isocarboxazid, Tranylcypramin, Chlorpromazin, Isoniazid, Trimethoprim, Cimetidin, Metiamid, Tryptamin, Clonidin, Metronidazol, Tyramin, Cyanid, Nazlinin (Alkaloid), Diamine (auch Histamin) und Nt-Methylhistamin.

In der WO 02/43745 wird die systemische Verwendung von DAO pflanzlichen Ursprungs zur Behandlung von Histamin-vermittelten Erkrankungen offenbart. Die Verabreichung von DAO oder von Enzymen allgemein, die aus Pflanzen direkt isoliert werden, ist aufgrund des häufigen Vorkommens von Allergenen in Pflanzen sehr problematisch, vor allem in Anbetracht der Tatsache, dass die in der WO 02/43745 offenbarten Hülsenfrüchte ein starkes allergenes Potential aufweisen.

Die US 3 639 579 A betrifft eine pharmazeutische Zubereitung, die unter anderem Diaminooxidase enthalten kann, zur Behandlung von Hämorrhoiden. In dieser Schrift wird Diaminooxidase in Form einer Salbe appliziert.

In der FR 2 215 944 A wird die Verwendung von pharmazeutischen Zusammensetzungen, welche Histaminase enthalten, zur Behandlung von allergischer Rhinitis, Asthma, Ekzemen und weiteren allergischen Reaktionen geoffenbart. Diese Zusammensetzungen werden dabei mittels Injektionen intramuskulär verabreicht. Im Zuge der Herstellung des Produkts gemäß der FR 2 215 944 A wird Diaminooxidase aus Plazentamaterial gewonnen, wobei vor Sterilfiltrierung und Lyophilisierung PEG 6000 (Polyethylenglykol 6000) hinzugegeben wird. Das hinzugegebene PEG 6000, das im Zuge der Lyophilisierung dazugegeben wird, dient dazu, das Enzym beim Gefrieren und Trocknen zu stabilisieren.

In der US 2004/115189 A1 ist die Verwendung von Diaminooxidase pflanzlichen Ursprungs zur Behandlung von Histamin-induzierten Krankheiten, wie beispielsweise von allergischem Schock, septischem Schock, allergischem Asthma und dergleichen, geoffenbart. Dabei ist die Anwendung in allen geoffenbarten Beispielen auf die systemische Applikation (parenteral oder inhalativ) beschränkt.

Die US 3 721 733 A betrifft pharmazeutische Zusammensetzungen, die Histaminase in Kombination mit anti-mikrobiellen Substanzen umfassen. Dabei dient die Zugabe von Histaminase zur Reduktion von Nebenwirkungen, die durch die Verabreichung von Antibiotika hervorgerufen werden.

In der US 5 270 033 A wird eine Zahnpasta (umfassend beispielsweise Glycerin, Sorbit usw.) geoffenbart, die unter anderem Enzyme, wie Oxidoreduktasen, insbesondere Diaminooxidase, und deren Substrate umfassen kann. Das durch die Reaktion der Oxidoreduktasen mit den Substraten entstehende Wasserstoffperoxid bewirkt einen kosmetischen Effekt an den Zähnen.

In der FR 2 101 095 A ist eine pharmazeutische Zubereitung zur Behandlung von Krankheiten, die durch Hyperhistaminämie bewirkt sind, geoffenbart. Eine derartige Zusammensetzung umfasst Diaminooxidase, welche aus humaner Plazenta gewonnen wird und in der Lage ist, Histamin, Putrescin und Cadaverin nicht aber Phenylethylamin und Tyramin zu oxidieren.

Aufgabe der vorliegenden Erfindung ist es, Zusammensetzungen zur Behandlung und zur Prävention von Histamin-induzierten Krankheiten und Krankheitszuständen zur Verfügung zu stellen, die die Nebenwirkungen der am Markt befindlichen Produkte, die vorwiegend Cortison umfassen, nicht aufweisen und die Nachteile des oben angeführten Standes der Technik beseitigen.

Daher betrifft die vorliegende Erfindung pharmazeutische und kosmetische Zusammensetzungen, umfassend Diaminooxidase, wobei die Zusammensetzung als Hydrogel vorliegt. Die Diaminooxidase liegt in den erfindungsgemäßen Zusammensetzungen im Wesentlichen in dessen aktiver Form vor, d.h. dass Enzyme, die beispielsweise kein Kupfer in deren prosthetischen Gruppe aufweisen und nicht zumindest Wild-Typ Aktivität zeigen, nicht für die erfindungsgemäßen Verwendungen geeignet sind.

Die erfindungsgemäße pharmazeutische Zusammensetzung weist eine Darreichungsform auf, die eine epidermale Verabreichung ermöglicht. Die epidermale Verabreichung von DAO ist vor allem bei Histamin-induzierten Krankheiten bzw. Krankheitszuständen an der Hautoberfläche bzw. an den äußersten Schichten der Haut von Vorteil, denn dadurch können beispielsweise allergische Reaktionen resultierend aus einem Kontakt der Haut mit einem Allergen erfolgreich behandelt werden. Ebenso wird Juckreiz, der bei verschiedenen Krankheiten, wie Urticaria, atopisches Ekzem und dgl. durch Histamin-Freisetzung ausgelöst wird, unterbunden.

Die pharmazeutische Zusammensetzung wird als Hydrogel zur Verfügung gestellt. Gemäß der vorliegenden Erfindung ist es möglich, DAO durch entsprechend den im Stand der Technik bekannten Aufbereitungsmethoden in pharmazeutischen Darreichungsformen zu überführen. Daher enthalten pharmazeutische Zusammensetzungen, die DAO beinhalten, auch weitere Inhaltstoffe, die verwendet werden, um einerseits das Enzym zu stabilisieren und andererseits das Enzym in die entsprechende galenische Form zu bringen. DAO kann natürlich auch mit anderen pharmazeutisch aktiven Wirkstoffen gemeinsam in einer einzigen Darreichungsform oder getrennt verabreicht werden, sofern das Enzym nicht durch einen dieser Wirkstoffe dermaßen inhibiert wird, dass eine Aktivität des Enzyms nicht die gewünschte Wirkung entfaltet.

Die erfindungsgemäßen Hydrogel-Zusammensetzungen umfassend DAO weisen vorzugsweise eine Viskosität von 0,5 bis 5 cp (centipoise), noch mehr bevorzugt von 1 bis 2 cp, insbesondere 1,1 bis 1,6 cp, bei einer Schergeschwindigkeit von 41 sec⁻¹ auf, wobei die Messung mit im Stand der Technik bekannten Viskosimetern erfolgen kann. Es hat sich herausgestellt, dass sich besonders in diesen Viskosiätsbereichen die DAO-hältige Hydrogel-Zusammensetzung vorteilhafte Eigenschaften (z.B. topische Verteilbarkeit, gute Handhabung) aufweist. Bei einer geringeren Viskosität von 0,5 cp erwies sich die Hydrogel-Zusammensetzung als zu dünnflüssig um eine anwenderfreundliche Handhabung des Präparats zu ermöglichen. Vorzugsweise werden als Gelbildner Polyacrylate (z.B. Carbopol), Zellulosederivate bzw. modifizierte Zellulose, insbesondere Hydroxyethylcellulose (z.B. Natrosol), Metylzellulose, Hydroxypropylmethylzellulose und Hydroxymethylzellulose, Stärke und modifizierte Stärke, natürliche und synthetische Gummis, wie z.B. Tragacanth, Guar, Carrageenan, Gelatine, Natriumalginat, PVP, Polyvinylalkohol und Mischungen davon. Besonders bevorzugt kommt als Gelbildner Hydroxyethylcellulose (z.B. Natrosol) zum Einsatz, wobei insbesondere Natrosol Typ 250 HHR bereits bei niedriger Konzentration besonders gute Stabilität aufweist. Der pH-Wert der Hydrogel-Zusammensetzung beträgt vorzugsweise 7,0 bis 9,0, vorzugsweise 7,2 bis 8,5, noch mehr bevorzugt von 7,5 bis 8,0. Dieser pH-Wertebereich wird vorzugsweise mit einem Puffer, insbesondere einem Bis/Tris-Puffer, eingestellt, wobei zur Aufrechterhaltung der DAO-Enzymaktivität und -Stabilität weitere Salze (z.B. 20-300 mM, vorzugsweise 50 bis 200 mM, insbesondere 100 mM, NaCl) zugegeben werden können. Ist die Gesamtkonzentration der Salze unter 20 mM weist die DAO eine schlechtere Stabilität auf, die zu einem Aktivitätsverlust führen kann.

Weiters können in der erfindungsgemäßen Hydrogel-Zusammensetzung auch Konservierungsmittel zugesetzt werden, die die Vermehrung von Mikroorganismen in der Zusammensetzung im Wesentlichen verhindert. Wichtig ist dabei die Konservierungsmittel so auszuwählen, dass die antimikrobiellen Substanzen die DAO-Aktivität nicht in einer Weise inhibiert, dass deren Enzymaktivität nicht mehr ausreicht, das Ziel der vorliegenden Erfindung (Abbau von Histamin) zu erreichen. Durch Aktivitätstests, die mit DAO und dem Konservierunsgmittel durchgeführt werden, können derartige Substanzen identifiziert werden. Vorzugsweise werden Chlorhexidin, Parabene (Para-Hydroxy-Benzoesäureester, insbesondere Butyl-, Ethyl-, Methyl- oder Propylparabene), Benzoate (z.B. Na-Benzoat), Sorbate (z.B. K-Sorbat), Carbamate (z.B. Iodopropynylbutylcarbamat) und Kombinationen davon (z.B. Rokosonal = Mischung aus Na-Benzoat, K-Sorbat und Iodopropynylbutylcarbamat) als Konservierungsmittel eingesetzt. Besonders bevorzugt sind Hydrogel-Zusammensetzungen, die Natrosol als Gelbildner und Rokosonal und/oder Parabene als Konservierungsmittel umfassen. Selbstverständlich können erfindungsgemäß auch Duft- und Aromastoffe, die beispielsweise in der kosmetischen Industrie eingesetzt werden, zugesetzt werden.

Ein weiterer Aspekt der vorliegenden Erfindung betrifft eine kosmetische Zusammensetzung, umfassend Diaminooxidase, welche als Hydrogel vorliegt. Bei erhöhter Histaminausschüttung bzw. bei Kontakt mit allergenen Stoffen (z.B. bei Kontaktallergien oder Neurodermitis) kann es zu Körperreaktionen an sichtbaren Stellen des Körpers kommen, die durch die Verabreichung von DAO in kosmetischen Zusammensetzungen unterdrückt werden können. Kosmetische Zusammensetzungen, enthaltend DAO, können weiters andere im Stand der Technik bekannte Inhaltsstoffe aufweisen, die in der Herstellung von kosmetischen Produkten verwendet werden. Kosmetische Zusammensetzunge, die Hydrogel umfassen, weisen im Wesentlichen dieselben Eigenschaften bzw. enthalten im Wesentlichen dieselben Inhaltsstoffe wie Hydrogele einer erfindungsgemäßen pharmazeutischen Zusammensetzung.

Vorzugsweise ist die Diaminooxidase, die in den erfindungsgemäßen Zusammensetzungen Verwendung findet, nicht-pflanzlichen Urpsrungs.

Die Verwendung von DAO nicht pflanzlichen Ursprungs in pharmazeutischen und kosmetischen Zusammensetzungen sowie Nahrungsergänzungsmitteln und diätetischen Lebensmitteln hat den Vorteil, dass in Pflanzen vorkommende Allergene die Verabreichung von DAO nicht negativ beeinflusst, da Allergene die körpereigene Histaminausschüttung wesentlich fördern. Es ist bekannt, dass vor allem Pflanzenstoffe für Histamin-induzierte Krankheiten verantwortlich sind. Die vollständige Entfernung allergieauslösender Inhaltstoffe aus einer DAO-Präparation pflanzlichen Ursprungs ist nur mit hohem präparativen Aufwand möglich, hingegen ist die erfindungsgemäße DAO, welche einen nicht pflanzlichen Ursprung aufweist gänzlich frei von derartigen pflanzlichen Allergenen.

Gemäß der vorliegenden Erfindung werden unter "nicht pflanzlichen Ursprung" alle DAO umfasst, die nicht aus Pflanzen sondern aus tierischen Organismen oder sonstigen nicht pflanzlichen Organismen gewonnen werden. Des Weiteren fallen erfindungsgemäß unter diese Definition alle DAO, die rekombinant in Zellkulturen (tierischen, bakteriellen, Hefen und dgl.) bzw. in nicht pflanzlichen Organismen jeglicher Art hergestellt werden, wobei die DNA für die rekombinant hergestellte DAO von pflanzlichen und/oder tierischen Organismen mittels im Stand der Technik bekannten Methoden isoliert und in Expressionssysteme kloniert und exprimiert wird.

Vorzugsweise umfassen alle in der vorliegenden Erfindung offenbarten Zusammensetzungen Diaminooxidase tierischen Ursprungs. Durch die Verwendung von tierischem DAO ist es möglich, dem menschlichen bzw. tierischen Körper ein Enzym zuzuführen, das dem von diesen Individuen selbst hergestellten Enzym bezüglich Glykosilierung, Aktivität und Spezifität der vom diesen Individuum selbst produzierten DAO sehr nahe kommt. Weiters ist es möglich pflanzliche Allergene zur Gänze von der Herstellung von DAO auszuschließen.

Vorzugsweise wird die Diaminooxidase aus Schweinenieren gewonnen. Schweinenieren zeichnen sich vor allem durch ihren hohen Gehalt an DAO aus. Aus Schweineniere kann das Enzym in einfacher Weise mit den im Stand der Technik bekannten Methoden isoliert werden.

Gemäß einer weiteren bevorzugten Ausführungsform umfassen die erfindungsgemäßen Zusammensetzungen Diaminooxidase rekombinanten Ursprungs. Durch die rekombinante Herstellung von DAO ist es möglich, große Mengen an Enzym herzustellen und dieses Enzym in einer hohen Ausbeute zu reinigen.

Vorzugsweise wird die rekombinante Diaminooxidase in prokaryotischen, vorzugsweise in bakteriellen, oder eukaryotischen, vorzugsweise in tierischen oder Hefe-, Zellkulturen exprimiert und aus den oben genannten Expressionssystemen isoliert. Die Aufreinigung mittels dieser Expressionssysteme hergestellten DAO, die entweder in den Zellen exprimiert oder aus den Zellen während der Expression ausgeschleust wird, erfolgt durch die im Stand der Technik bekannten Methoden. Dabei ist es auch möglich die DAO mit einer Peptid- (z.B. His-Tag), Polypeptid- oder Proteinsequenz (z.B. GST-Tag) zu versehen um die Aufreinigung zu vereinfachen. Die rekombinante DAO kann weiters durch gentechnische Verfahren derart modifiziert sein, dass die Enzymaktivität dieser DAO die Enzymaktivität der Wild-Typ DAO übertrifft.

Die erfindungsgemäße Diaminooxidase kann zur Herstellung eines Arzneimittels zur Behandlung von Histamin-induzierten Krankheitsbildern verwendet werden. DAO ist bekannterweise für den Abbau von Histamin im menschlichen und tierischen Körper verantwortlich. Da Histamin-induzierte Krankheiten durch einen Überschuss von Histamin, das aufgrund eines Mangels an Diaminooxidase, durch Inhibition von DAO oder durch einen Histaminüberschuss, der in der Regel durch Nahrungsmittel, aber auch durch weitere extrinsische Faktoren, wie z.B. Kontakt mit Allergenen, hervorgerufen werden, eignet sich die Verabreichung von erfindungsgemäßer DAO zur Behandlung dieser Krankheiten bzw. Krankheitsbilder.

Ein weiterer Aspekt der vorliegenden Erfindung betrifft die Verwendung der erfindungsgemäßen Diaminooxidase zur Herstellung eines Arzneimittels zur Behandlung von Urticaria, insbesondere von chronischer und akuter Urticaria. Bei Urticaria kommt es bei Freisetzung von Histamin zu einer Erweiterung von Venolen und zur überhöhten Durchlässigkeit der Kapillaren mit daraus resultierendem Ödem. Durch die Verabreichung von DAO an den betroffenen Hautzonen ist es möglich, das Histamin an betroffenen Stellen abzubauen und dadurch den Juckreiz der Urticaria zu unterbinden.

Ein weiterer Aspekt der vorliegenden Erfindung betrifft die Verwendung von erfindungsgemäßer Diaminooxdiase zur Herstellung eines Arzneimittels zur Behandlung von Kontaktallergie. Kontaktallergien werden durch Substanzen (Allergene), die durch Eindringen in die Haut allergische Reaktionen auslösen, hervorgerufen. Um die durch diesen Kontakt ausgeschütteten Histamine abzubauen und somit die Histamin-induzierenden Krankheitserscheinungen zu unterbinden bzw. zu lindern, wird DAO verabreicht.

Gemäß einem weiteren Aspekt der vorliegenden Erfindung wird eine erfindungsgemäße Diaminooxidase zur Herstellung eines Arzneimittels zur Behandlung von atopischer Dermatitis verwendet. Atopische Dermatitis, auch bekannt unter dem Namen Neurodermitis, ist eine häufige Hauterkrankung mit starkem Juckreiz zumeist bei Kindern und jugendlichen Erwachsenen. Ursache dieses Juckreizes ist die übermäßige Ausschüttung von Histaminen an den betreffenden Hautstellen. Auch in diesem Fall kann DAO zur Reduktion der Histamine in diesen Bereichen beitragen und somit die Symptome von atopischer Dermatitis lindern bzw. verhindern.

Die Erfindung wird weiters durch folgende Beispiele erläutert, ohne jedoch auf diese beschränkt zu sein.

### BEISPIELE:

### Beispiel 1

Weiters wurde das Enzym in einem Hydrogel stabilisiert. Lagerung bei Raumtemperatur und 37°C zeigten keinen Abfall der Aktivität innerhalb von 4 Monaten. Auftretende Bläschen und Juckreiz an der Haut nach Stimulation mit Histamin verschwanden wenige Minuten nach Applikation des Hydrogels.

### Beispiel 2

Die in Hydrogel stabilisierte DAO wurde insgesamt an 13 Personen getestet. Anhand eines Fragebogens wurde unterschieden zwischen Belastung durch Kontakallergie (n=5), Neurodermitis/Dermatosen (n=6) und Insektenstichen (n=2). Bei allen Kontaktallergikern und Neurodermitis-Patienten wurde eine positive Wirkung attestiert, bei Insektenstichen konnten 50% der Probanden von einer positiven Wirkung berichten.

| ***Belastung*** | ***Positive Wirkung*/*Anzahl Probanden*** |
|---|---|
| Neurodermitis/Dermatose | 6/6 |
| Kontaktallergie | 5/5 |
| Insektenstich | 1/2 |

Die Wirkung des Hydrogels trat innerhalb der ersten 10 Minuten ein, wobei die Wirkung durchschnittlich länger als eine Stunde anhielt.

| | ***0-10 min*** | ***10-30 min*** | ***30-60 min*** | ***> 1h*** | ***> 3h*** |
|---|---|---|---|---|---|
| Wirkungsbeginn | 11 | 1 | | | |
| Wirkungsdauer | | 3 | 1 | 2 | 6 |

Kein Proband meldete unangenehme Effekte bei wiederholter Anwendung.

Zwei Neurodermitis-Patienten gaben an, dass das erfindungsgemäße Hydrogel besser als eine aktuell eingesetzte Cortison-Salbe wirkt.

## Patentansprüche

1. Pharmazeutische Zusammensetzung umfassend Diaminooxidase, **dadurch gekennzeichnet, dass** die Zusammensetzung als Hydrogel vorliegt.

2. Kosmetische Zusammensetzung, umfassend Diaminooxidase, **dadurch gekennzeichnet, dass** die Zusammensetzung als Hydrogel vorliegt.

3. Zusammensetzung nach Anspruch 1 oder 2, **dadurch gekennzeichnet, dass** die Diaminooxidase nicht-pflanzlichen Ursprungs ist.

4. Zusammensetzung nach Anspruch 3, **dadurch gekennzeichnet, dass** die Zusammensetzung Diaminooxidase tierischen Ursprungs umfasst.

5. Zusammensetzung nach Anspruch 4, **dadurch gekennzeichnet, dass** die Diaminooxidase aus Schweinenieren stammt.

6. Zusammensetzung nach einem der Ansprüche 1 bis 5, **dadurch gekennzeichnet, dass** die Zusammensetzung Diaminooxidase rekombinanten Ursprungs umfasst.

7. Zusammensetzung nach Anspruch 6, **dadurch gekennzeichnet, dass** die Zusammensetzung rekombinante Diaminooxidase gewonnen aus prokaryotischen, vorzugsweise aus bakteriellen, oder aus eukaryotischen, vorzugsweise aus tierischen oder Hefe-, Zellkulturen umfasst.

8. Verwendung von Diaminooxidase zur Herstellung eines Arzneimittels zur Behandlung von Urticaria, insbesondere von chronischer und akuter Urticaria, **dadurch gekennzeichnet, dass** das Arzneimittel als Hydrogel vorliegt.

9. Verwendung von Diaminooxidase zur Herstellung eines Arzneimittels zur Behandlung von Kontaktallergie, **dadurch gekennzeichnet, dass** das Arzneimittel als Hydrogel vorliegt.

10. Verwendung von Diaminooxidase zur Herstellung eines Arzneimittels zur Behandlung von atopischer Dermatitis, **dadurch gekennzeichnet, dass** das Arzneimittel als Hydrogel vorliegt.

## Claims

1. Pharmaceutical composition comprising diaminooxidase, **characterised in that** the composition is present in the form of hydrogel.

2. Cosmetic composition comprising diaminoxidase, **characterised in that** the composition is present in the form of hydrogel

3. Composition according to claim 1 or 2, **characterised in that** the diaminooxidase is of non-plant origin.

4. Composition according to claim 3, **characterised in that** the diaminooxidase is of animal origin.

5. Composition according to claim 4, **characterised in that** the diaminooxidase originates from porcine kidneys.

6. Composition according to any one of claims 1 to 5, **characterised in that** the composition comprises diaminooxidase of recombinant origin.

7. Composition according to claim 6, **characterised in that** the composition comprises recombinant diaminooxidase obtained from prokaryotic, preferably from bacterial, or from eukaryotic, preferably from animal or yeast, cell cultures.

8. Use of diaminooxidase for the production of a medicinal product for the treatment of urticaria, more particularly chronic and acute urticaria, **characterised in that** the medicinal product is present in the form of hydrogel.

9. Use of diaminooxidase for the production of a medicinal product for the treatment of contact allergy, **characterised in that** the medicinal product is present in the form of hydrogel.

10. Use of diaminooxidase for the production of a medicinal product for the treatment of atopic dermatitis, **characterised in that** the product is present in the form of hydrogel.

## Revendications

1. Composition pharmaceutique comprenant de la diamine-oxydase, **caractérisée en ce que** la composition se présente sous forme d'hydrogel.

2. Composition cosmétique comprenant de la diamine-oxydase, **caractérisée en ce que** la composition se présente sous forme d'hydrogel.

3. Composition selon la revendication 1 ou 2, **caractérisée en ce que** la diamine-oxydase est d'origine non végétale.

4. Composition selon la revendication 3, **caractérisée en ce que** la composition comprend de la diamine-oxydase d'origine animale.

5. Composition selon la revendication 4, **caractérisée en ce que** la diamine-oxydase provient de reins porcins.

6. Composition selon l'une des revendications 1 à 5, **caractérisée en ce que** la composition comprend de la diamine-oxydase provenant de recombinaison génétique.

7. Composition selon la revendication 6, **caractérisée en ce que** la composition comprend de la diamine-oxydase recombinée obtenue à partir de cultures cellulaires procaryotes, de préférence bactériennes, ou de cultures cellulaires eurocaryotes, de préférence animales ou de levure.

8. Utilisation de diamine-oxydase pour la préparation d'un médicament destiné au traitement de l'urticaire, notamment de l'urticaire chronique et aigue, **caractérisée en ce que** le médicament se présente sous forme d'hydrogel.

9. Utilisation de diamine-oxydase pour la préparation d'un médicament destiné au traitement de l'allergie de contact, **caractérisée en ce que** le médicament se présente sous forme d'hydrogel.

10. Utilisation de diamine-oxydase pour la préparation d'un médicament destiné au traitement de la dermatite atopique, **caractérisée en ce que** le médicament se présente sous forme d'hydrogel.
